# EUROPEAN PATENT APPLICATION

(11) **EP 2 915 500 A1**
(43) Date of publication of application: **09.09.2015**
(21) Application number: 15152990.6
(22) Date of filing: 29.01.2015
(51) Int. Cl.: A61B 18/20, A61N 5/06

(54) **A multi-wavelength laser device for skin treatment**

(30) Priority: 07.03.2014 US 201461949274 P
(71) Applicant: Syneron Medical Ltd., 20692 Yoqneam Illit (IL)
(72) Inventor: Huppert, Avram, 4035514 Kfar Yona (IL); Israeli, Roy, 3455541 Haifa (IL); Eisenmann, Shmuel, 3713405 Pardes Chana-Karkur (IL)
(74) Representative: Jennings, Tara Romaine

(57) **Abstract**

Disclosed is a handpiece for skin treatment that includes at least two laser diode bars, with each bar configured to emit a laser beam with a different wavelength.

## Description

### TECHNOLOGY FIELD

The laser diodes stack relates generally to a device for skin treatment using electromagnetic radiation. More particularly, the laser diodes stack relates to a device including a number of solid state lasers operating at different wavelengths produced as one monolithic device.

### BACKGROUND

Lasers are widely used in dermatological skin applications such as hair removal, removal of pigmented lesions, tattoos, vascular lesions, wrinkles, acne, and skin tightening. Dermatological laser treatments are typically based on selective targeting of a chromophore in the skin by an appropriate choice of wavelength and pulse duration of the laser light. Different skin deficiencies could include different chromophores, accordingly, the products for skin treatment have to include a plurality of different sources providing a variety of wavelengths. It has been proven that lasers typically provide better treatment results than broadband light sources. However, laser devices typically provide a single wavelength of light. Because of this, most of the skin treatment products include a number of lasers. Inclusion of several different lasers into one product increases the complexity and cost of the product.

The lasers typically used for skin treatment are Alexandrite lasers, Nd:YAG lasers; Erbium lasers and different gas lasers. Each laser has its individual power supply, control and optics. The power supply is typically located in a separate housing. The optics, that could be a fiber optics light guide or an articulated arm, generally directs a laser beam that includes different wavelengths to a handpiece that the caregiver applies to the treated segment of the skin. A cable that includes the fiber optics light guide and some electrical cabling reduces the ease of handling of the handpiece and caregiver freedom.

### BRIEF SUMMARY

Disclosed is a handpiece for skin treatment that includes at least two laser sources such as diode bars, with each source configured to emit a laser beam with a different wavelength. The handpiece also includes a wavelength combining device, the wavelength combining device configured to receive the at least first and second wavelength and combine them into a homogenized laser beam containing the at least first and second wavelength. A controller device could be included in the handpiece or the machine that the handpiece is connected to. The controller device is configured to control at least the first and second laser diode bars and in particular the power that each of the laser diode bars emit. Each of the laser diode bars could be individually addressed and controlled. The amount of power for each of the wavelength will be adapted according to the treated skin type (i.e. amount of melanin in the epidermis) and the target tissue type, location and absorption spectrum in order to get the safest and the highest efficacy in treating that targeted skin or tissue.

The homogenized laser beam includes at least two wavelengths selected from a plurality of wavelengths in the range of 532-1250nm and 1400-1600nm.

For skin treatment the handpiece is applied to a segment of skin to be treated and operated to irradiate the treated skin segment by a homogenized laser beam containing the first and the second wavelength. Following application of the homogenized laser beam to the treated skin segment the handpiece could be repositioned to treat a next skin segment.

Disclosed herein is a laser diode bars stack assembly comprising:
at least one laser diode bar configured to emit a laser beam with a first wavelength;
at least one laser diode bar configured to emit a laser beam with a second wavelength;
a wavelength combining device, the wavelength combining device is configured to receive the first and the second wavelength and combine them into a homogenized laser beam containing the first and the second wavelength.

Optionally, the laser diode bars are mounted on a common substrate and wherein the substrate serves as a heat sink.

Optionally, the laser diode bars stack assembly further comprises a controller device configured to control at least a first and a second laser diode bars and wherein each of the laser diode bars is individually addressed and controlled.

Optionally, each of the at least two semiconductor laser bars emits at least at one of the wavelengths of 775nm, 810nm, 905nm, 1064nm, 1400nm and 1600nm.

Optionally, the wavelength combining device is one of a group consisting of a light guide, a prism beam combiner, and a reflective beam combiner.

Optionally, the wavelength combining device is at least one of a group consisting of a reflective optics and refractive optics.

Further optionally, the wavelength combining device is at least one optical fiber.

Also disclosed herein is a handpiece for skin treatment comprising
at least two laser diode bars, with each bar configured to emit a laser beam with a different wavelength;
a wavelength combining device, the wavelength combining device configured to receive at least a first and a second wavelength and combine them into a homogenized laser radiation beam containing at least the first and second wavelength.

Optionally, the handpiece further comprises a controller device configured to control at least a first and a second laser diode bars and where in each of the laser diode bars is individually addressed and controlled.

Optionally, the homogenized laser radiation beam includes at least two wavelengths selected from a group of wavelengths consisting of 775nm, 810nm, 905nm, 1064nm, 1400nm and 1600nm.

Optionally, the wavelength combining device is one of a group consisting of a light guide, a prism beam combiner, and a reflective beam combiner.

Further optionally, the wavelength combining device is at least one optical fiber.

Also disclosed herein is a cosmetic method of skin treatment comprising:
applying to a segment of skin to be treated a handpiece including at least two laser diode bars, with each bar configured to emit a laser radiation with a different wavelength; and
a wavelength combining device, the wavelength combining device configured to receive laser radiation with different wavelengths and combine them into a homogenized laser beam containing different wavelengths;
operating the handpiece to irradiate the treated skin segment by a homogenized laser beam containing the different wavelengths; and
following application of homogenized laser beam to the treated skin segment reposition the handpiece to treat a next skin segment.

Also disclosed herein is a cosmetic method of skin treatment comprising:
applying to a segment of skin to be treated a handpiece including at least two laser diode bars, with each bar configured to emit a laser radiation with a different wavelength; and
a wavelength combining device, the wavelength combining device configured to receive laser radiation with different wavelengths and combine them into a homogenized laser beam containing different wavelengths;
controlling the amount of energy in each wavelength according to the skin type and target chromophores to be treated; and
operating the handpiece to irradiate the treated skin segment by a homogenized laser beam containing the different wavelengths; and
following application of homogenized laser beam to the treated skin segment and repositioning the handpiece to treat a next skin segment.

Optionally, the wavelength combining device is receiving laser radiation with different wavelengths and combines the different wavelengths into a homogenized laser beam containing different wavelengths.

Optionally, a controller is controlling the amount of energy in each wavelength according to the skin type and target chromophores to be treated.

Optionally, the amount of power for each of the wavelength is adapted according to the skin type to be treated.

### BRIEF DESCRIPTION OF THE DRAWINGS

The features and advantages of the present handpiece will become apparent from the following detailed description which is to be read in conjunction with the accompanying drawings.
FIG. 1 is a simplified example of an existing laser diode array or bar;
FIG. 2 is a simplified example of an existing laser diodes stack;
FIGS. 3A and 3B are simplified illustrations of a laser diode bars assembly according to an example;
FIGS. 4A and 4B are examples of laser beam combiners configured to combine and homogenize different wavelengths emitted by laser diode bars;
FIG. 5 is a schematic illustration of an example of a laser diodes stack including four laser diode bars with each laser diode bar emitting a different wavelength; and

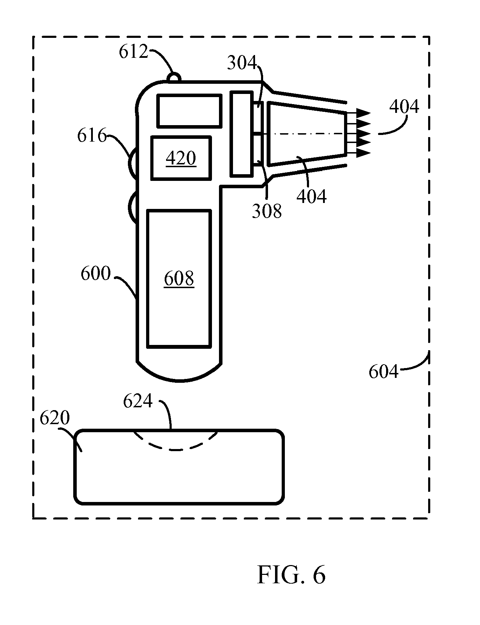

FIG. 6 is a schematic illustration of an example of a handpiece of a skin treatment apparatus employing the present laser diodes stack.

### DETAILED DESCRIPTION

Semiconductor lasers are available in a variety of wavelengths and could be used as substitutes for Alexandrite, Nd:YAG, Erbium, and other similar solid state or gas lasers. However, each semiconductor laser also emits in a relatively narrow wavelength range and requires a dedicated optical system for combining the different wavelength into one laser beam that could be used for skin treatment. The current device is a stack of semiconductor laser arrays such as laser diode arrays or bars that generates and combines two or more wavelengths into one laser beam suitable for skin treatment.

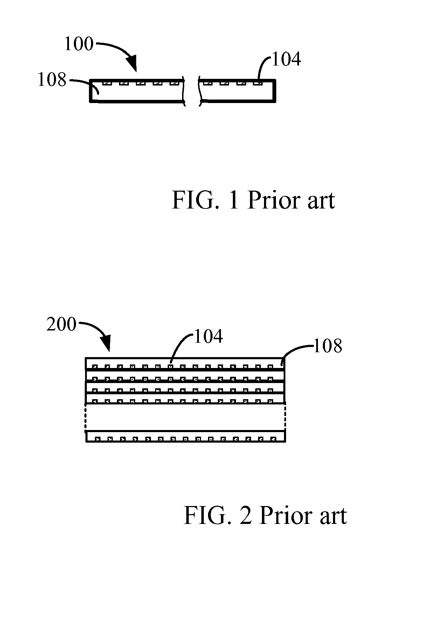

FIG. 1 is a simplified example of an existing laser diode array or bar. A laser diode bar 100 is typically a one-dimensional array of a plurality of individual semiconductor laser emitters or laser diodes 104 assembled on a common substrate 108. A laser diode array or bar could include 20 - 50 - 70 or more individual laser diodes 104 emitting the same wavelength. As shown in FIG. 2, a number of laser diode bars could be stacked to form what is termed as a laser diode bars stack 200. Different optical arrangements could be used to couple the laser beams emitted by each of the individual laser diodes into one high power laser beam. Existing laser diode bar stacks do not support inclusion of laser diode bars emitting different wavelength into one laser diode stack. The current disclosure provides a method and device supporting manufacture and coupling of laser diode stacks emitting different wavelengths into one common laser beam. Availability of a laser diode stack emitting different wavelengths into one common laser beam could support miniaturization of skin treatment equipment and simplify the use of such equipment.

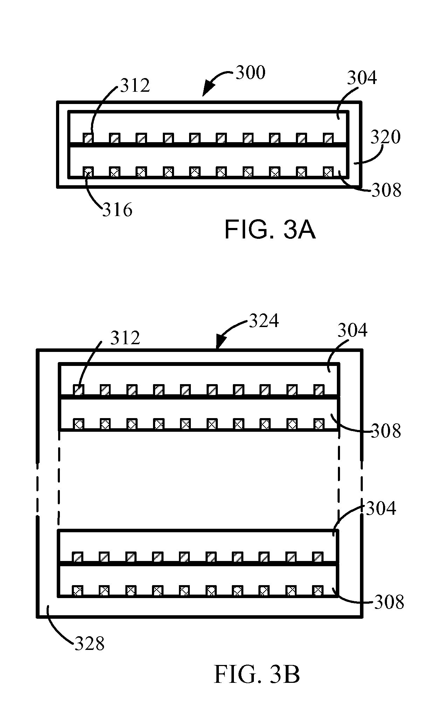

In one example illustrated in FIG. 3A, a laser diode bars assembly or stack 300 includes two laser diode bars 304 and 308. Each of two laser diode bars 304 and 308 includes laser emitters 312 and 316 emitting laser radiation at a different wavelength. For example, laser diode bar 304 could be configured to emit a laser radiation with a first wavelength and laser diode bar 308 could be configured to emit a laser radiation with a second wavelength. The wavelength could be such as 760nm, 775nm, 810nm, 905nm, 1064nm, 1210nm, 1470nm, 1540nm and other wavelengths. Laser diode bars 304 and 308 could be mounted on a common substrate or mount 320 that could be made of a suitable heat conducting material such as aluminum or copper and serve as heat sink for the laser diode bars stack or assembly. In some examples, laser diode bars assembly 324 could include more than two laser diode bars. For example, there could be four or ten, or more laser diode bars 304 emitting at a first wavelength and four or ten, or more laser diode bars 308 emitting at a second wavelength. Each laser diodes bar could emit between 10W to 120W of laser power. The plurality of laser diode bars could be mounted on a common substrate 328 that would serve as a mount and as a heat sink.

Laser diode bars assembly or stack 300 (or 324) could also include a coupling optics that could be a refractive or reflective optics. For example, an optical beam combiner could be configured to combine and homogenize the two different wavelengths emitted by laser diode bars 304 and 308 into a common laser beam 404. In one example, the beam combiner could be a refractive light guide 408 that could be a tapered trapezoidal prism or other suitable cross section prisms or mirror systems. Light guide 408 could be made from sapphire or other suitable material and have dimensions of 25 to 100mm in length and width matching the size of the laser diode bars stack or assembly. The most widely spread width of the laser diode bars is about 10mm.

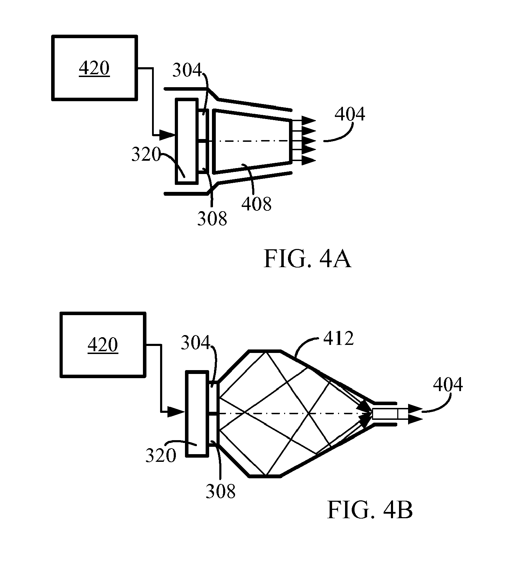

FIG. 4B illustrates a beam combiner 412 of reflective type. A combination or refractive optical elements such as prisms and reflective optical elements is also possible to use to combine laser radiation emitted by different laser diode bars. Optical fiber could also be used to combine and homogenize the two different wavelengths emitted by laser diode bars 304 and 308 into a common laser beam 404. Each laser diodes bar could be coupled into an optical filer and the individual optical fibers could be spliced into another optical fiber that could serve as a wavelength combining device and homogenize the wavelengths delivered by different spliced with it optical fibers.

The laser diode bars stack or assembly could also include a controller device 420 configured to control the first and the second laser diode bars and the whole stack. Controller 420 could be configured to address and control each laser diodes bars 304 or 308 individually as well as individual emitters within each of the laser diode bars. The amount of power for each of the wavelength emitted by the stack or even individual diode bar of each stack, will be adapted and controlled according to the treated skin type (i.e. amount of melanin in the epidermis) and the target tissue type, location and absorption spectrum in order to get the safest and the highest efficacy in treating that targeted skin or tissue. The amount of power for example could be 40% of the first wave length and 60% of the second wavelength. It could also 10% of the second wavelength and 90% of the first wavelength. The mix of wavelengths could be adapted for treatment of different hues of fair or dark skin as it could be determined by the amount of melanin in the epidermis. The adaptation of the power for each of the wavelength emitted by the laser bars or lasers could be manual or automatic in response for the melanin values entered by the operator.

In one example, the stack 300 of laser diode bars could be replaced by two or more fiber lasers or solid state lasers, which are coupled to a fiber or free space optics and are used to be delivered to the hand piece a mix of two or more wavelength with power of 10W to 10kW. Such lasers could for example be Alexandrite or Nd:YAG laser, Erbium lasers and different gas lasers. A frequency doubling device, such as a KTP plate (KTP is abbreviation for Potassium Titanyl Phosphate Single Crystal, (KTiOPO4)) or similar could be used to provide additional wavelengths. In some examples, angular orientation of the frequency doubling device could be used to vary and control the mix of power provided by the lasers.

In some examples, control of the amount of power in each of the wavelengths, regardless of the laser source type, is adding interference filters (band pass or high/low pass) designed for the wavelengths in use and misaligning them in order to achieve the needed energy in each of the used wavelengths.

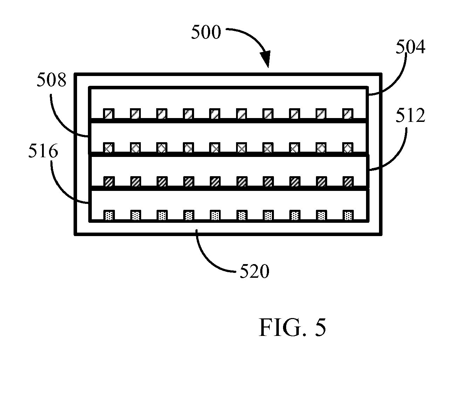

In some examples, the sources are at least two fiber lasers, each emtting in a different wavelength. Each of fiber laser sources could be spliced into another optical fiber that could serve as a wavelength combining device and homogenize the wavelengths delivered by different fiber lasers spliced with it optical fibers into a single fiber.
In some examples, as shown in FIG. 5, laser diode bars stack or assembly 500 could include more than two laser diode bars. For example, there could be four laser diode bars 504, 508, 512, and 516 with each of the laser diode bars emitting at a different wavelength. The plurality of laser diode bars 504, 508, 512, and 516 could be mounted on a common substrate 520 and have a common beam combiner and controller.

Because of the relatively small size of the laser diodes stack, the stack could be mounted within a handpiece 600 of a skin treatment apparatus 604. A rechargeable battery 608 could be included in handpiece 600 and configured to provide power to laser diode bars, controller 420 and different handpiece 600 status indicators such as one or more LEDs 612 or display. LEDs 612 could light in a number of different colors with each color indicating the current handpiece 600 status. One or more button switches 616 could be configured to switch ON or OFF different functions of the handpiece 600. For charging, handpiece 600 could be inserted into a docking and charging station 620 receptacle 624.For skin treatment handpiece 600 including at least two laser diode bars, with each bar configured to emit a laser radiation with a different wavelength and a wavelength combining device, could be applied to a segment of skin to be treated and operated to irradiate the segment of skin to be treated by a homogenized laser beam containing the first and the second wavelength and following application of homogenized laser beam to the treated skin segment repositioning the handpiece to treat a next skin segment. The amount of energy coupled into each wavelength could be set and controlled according to the skin type and target chromophores to be treated. Operating the handpiece to irradiate the treated skin segment by a homogenized laser beam containing the different wavelengths and following application of homogenized laser beam of proper power to the treated skin segment, repositioning the handpiece to treat a next skin segment.

Laser diode bars stack could be implemented as an exchangeable stack combining different wavelength combinations and could be inserted according to a desired skin treatment protocol. Two wavelengths simultaneously irradiating the skin segment to be treated could be selected for example to provide hair removal and wrinkle treatment or acne treatment and hair removal. For example, a combination of laser diode bars emitting at 760nm and 1064 or 808nm and 1064nm could be used for hair removal. Other combinations most appropriate for a desired treatment could be used. It should be recognized that a number of variations of the above-described examples will be obvious to one of ordinary skill. Accordingly, the apparatus and method are not to be limited by those specific examples and methods as shown and described herein. Rather, the scope of the apparatus and method is to be defined by the following claims and their equivalents.

## Claims

1. A handpiece for skin treatment comprising:
a laser diode bars stack assembly (300, 500) including at least two laser diode bars (304, 308), with each bar configured to emit a laser beam with a different wavelength;
a wavelength combining device (412), the wavelength combining device (412) configured to receive at least a first and a second wavelength and combine them into a common homogenized laser radiation beam (404) containing at least the first and second wavelength.

2. The handpiece according to claim 1 wherein the at least two laser diode bars (304, 308) are mounted on a common substrate (320, 520) and wherein the substrate (320) serves as a heat sink.

3. The handpiece according to claim 1 or claim 2, further comprising a controller device (420) configured to control at least a first (304), and a second (308), laser diode bars and wherein each of the laser diode bars is individually addressed and controlled.

4. The handpiece according to claim 3 wherein the controller device (420) is configured to control the amount of energy in each wavelength according to the target chromophores to be treated.

5. The handpiece according to any preceding claim, wherein each of the at least two semiconductor laser bars (304, 308), emits at least at one of the wavelengths of 775nm, 810nm, 905nm, 1064nm, 1400nm and 1600nm.

6. The handpiece according to any preceding claim, wherein the common homogenized laser radiation beam (404) includes at least two wavelengths selected from a group of wavelengths consisting of 775nm, 810nm, 905nm, 1064nm, 1400nm and 1600nm.

7. The handpiece according to any preceding claim, wherein the wavelength combining device (412) is one of a group consisting of a light guide, a prism beam combiner, and a reflective beam combiner.

8. The handpiece according to any preceding claim, wherein the wavelength combining device (412) is at least one optical fiber.
